# EUROPÄISCHE PATENTSCHRIFT

(11) **EP 3 313 565 B1**
(45) Veröffentlichungstag und Bekanntmachung des Hinweises auf die Patenterteilung: **14.08.2019**
(21) Anmeldenummer: 16731942.5
(22) Anmeldetag: 27.06.2016
(51) Int. Cl.: B01J 19/18, C07C 263/10

(54) **VERFAHREN ZUR HERSTELLUNG VON POLYISOCYANATEN**
METHOD FOR MANUFACTURING POLYISOCYANATES
PROCÉDÉ DESTINÉ À LA FABRICATION DE POLYISOCYANATES

(30) Priorität: 29.06.2015 EP 15174217
(43) Veröffentlichungstag der Anmeldung: 02.05.2018
(73) Patentinhaber: Covestro Deutschland AG, 51373 Leverkusen (DE)
(72) Erfinder: ARRAS, Jürgen, 25524 Itzehoe (DE); BUSCH, Jan, 40477 Düsseldorf (DE); STEFFENS, Christian, 51067 Köln (DE); WASTIAN, Dietmar, 41542 Dormagen (DE); KELLER-KILLEWALD, Manfred, 41539 Dormagen (DE); CONZELMANN, Holger, 71384 Weinstadt (DE)
(74) Vertreter: Levpat
(86) Internationale Anmeldenummer: PCT/EP2016/064811
(87) Internationale Veröffentlichungsnummer: WO 2017/001322

(56) Entgegenhaltungen:
- EP-A1- 1 867 632
- DE-A1- 2 112 181
- DE-A1- 10 260 082
- US-A- 4 128 569

## Beschreibung

Die vorliegende Erfindung betrifft ein kontinuierliches Verfahren zur Herstellung eines Polyisocyanats, bei dem ein Polyamin und Phosgen zunächst überwiegend zu Carbaminsäurechlorid und Aminhydrochlorid und nur in untergeordneten Anteilen zu Polyisocyanat umgesetzt werden, und ein Teil des so erhaltenen Carbaminsäurechlorid- und Aminhydrochlorid-haltigen Reaktionsgemisches in die Umsetzung mit Phosgen zurückgeführt wird, wobei Polyamin, Phosgen und das Carbaminsäurechlorid- und Aminhydrochlorid-haltige Reaktionsgemisch in einer Mischeinrichtung innig miteinander vermischt werden. Der nicht in die Phosgenierung zurückgeführte Teil des Carbaminsäurechlorid- und Aminhydrochlorid-haltigen Reaktionsgemisches wird zum Polyisocyanat aufgearbeitet.

Zur Herstellung von Polyisocyanaten durch Phosgenierung der korrespondierenden Amine sind viele Verfahren bekannt und in der Literatur beschrieben. Abhängig vom Typ der Amine kann die Reaktion in der Gas- oder Flüssigphase sowie diskontinuierlich oder kontinuierlich durchgeführt werden (W. Siefken, Liebigs Ann. 562, 75 - 106 (1949)).

Die Durchführung kontinuierlicher Synthesen von organischen Isocyanaten im großtechnischen Maßstab ist bereits mehrfach beschrieben worden, siehe beispielsweise Ullmanns Encyklopädie der technischen Chemie, 4. Aufl. (1977), Band 13, S. 351 bis 353. Es kommen dabei sowohl aromatische Isocyanate wie beispielsweise Methylendiphenyldiisocyanat (nachfolgend MMDI - "monomeres MDI"), Polymethylen-Polyphenylen-Polyisocyanat (ein Gemisch aus MMDI und dessen höheren Homologen, nachfolgend PMDI, "polymeres MDI") oder Toluylendiisocyanat (nachfolgend TDI) als auch aliphatische Isocyanate wie z. B. Hexamethylendiisocyanat (nachfolgend HDI) oder Isophorondiisocyanat (nachfolgend IPDI) weltweit zum Einsatz.

Nahezu ausschließlich in kontinuierlicher Fahrweise werden die industriellen Verfahren zur Produktion aromatischer Isocyanate wie MMDI, PMDI und TDI sowie aliphatischer Isocyanate wie HDI und IPDI betrieben. Als Beispiel für ein solches Verfahren in verschiedenen kontinuierlich geführten Kesseln sei DE-A-844 896 genannt.

Üblicherweise erfolgt die Phosgenierung primärer Amine (RNH₂) in Stufen, wobei zuerst bei niedriger Temperatur das Carbaminsäurechlorid (RNHCOCl) aus den Ausgangsstoffen gebildet wird und dieses anschließend bei erhöhter Temperatur in das entsprechende Isocyanat (RNCO) überführt wird, wobei in beiden Schritten Chlorwasserstoff abgespalten wird. Während der ersten Stufe, der sog. "Kaltphosgenierung", tritt als wesentliches Nebenprodukt das zu dem eingesetzten Amin korrespondierende Aminhydrochlorid ("RNH₂·HCl" = RNH₃Cl) auf, welches in der sog. "Heißphosgenierung" in Anwesenheit von Phosgen zum entsprechenden Isocyanat reagiert. Bei der Kaltphosgenierung werden üblicherweise Temperaturen unterhalb 60 °C angewendet, im Falle der Heißphosgenierung werden Temperaturen zwischen 100 °C und 200 °C erreicht. Zweistufige Verfahren werden beispielsweise in den Schriften DE-A-20 58 032, DE-A-21 53 268 und DE-A-1 233 854 erläutert.

Bei allen technisch angewandten Temperaturen und Drücken erfolgt die Reaktion zwischen Amin und Phosgen in der Flüssigphase sehr rasch. Um Nebenreaktionen zu vermeiden, sollte das Mischen der Reaktanden sehr effektiv durchgeführt werden. Die Phosgenierung von primären Aminen in einem Mischer-Reaktor als erster Stufe ist daher in vielen Veröffentlichungen gezeigt worden.

Mischer können in verschiedene Klassen unterteilt werden. Neben dynamischen (z. B. Rührer, Turbinen oder Rotor-Stator-Systeme) und statischen Mischern wie beispielsweise Kenics-, Schaschlik- oder SMV-Mischer sind auch Düsenmischer bekannt (Ind. Eng. Chem. Res. 26, 1987, 1184 - 1193). Beispielsweise eignen sich Stachelmischer (EP-A-2 077 150) und Lefos-Düsen (EP-A-0 322 647) besonders zur Herstellung aromatischer Isocyanate.

Eine Reihe von Apparaten wurde für die Phosgenierung von Aminen entwickelt, die optional auch als Phasentrennbehälter verwendet werden können. Die Phosgenierung von Aminen zu den entsprechenden Isocyanaten kann daher im Rührkessel (z. B. DE-OS 14 68 445), in einer Rührkesselkaskade (z. B. DE-PS 844 896) oder in Rohrreaktoren stattfinden, wobei letztere sowohl gepackt (z. B. WO-A-99/54289) als auch ungepackt (z. B. Ullmanns Encyklopädie der technischen Chemie, 4. Aufl. (1977), Band 13, S. 351 - 353) ausgeführt sein können. Bei vermindertem Reaktorvolumen können unter Rückführung auch Kreislaufreaktoren zum Einsatz kommen, um eine ausreichende Verweilzeit zur Vervollständigung der Reaktion zu gewährleisten.

Die Offenlegungsschrift DE-OS-1 593 412 beschreibt grundlegend ein kontinuierliches Herstellungsverfahren organischer Isocyanate, das als erste Reaktionsstufe einen "Reaktionskreis" (in der Zeichnung das in Form einer Ringleitung vorliegende Rohr 3) beinhaltet, dem sich in einer zweiten Reaktionsstufe eine destillative Aufarbeitung in einer Kolonne, in welcher das vorliegende Carbaminsäurechlorid zum entsprechenden Isocyanat umgesetzt wird, anschließt. Neben MMDI wurde auch die Herstellung von TDI und aliphatischen Isocyanate beschrieben. Im Reaktionskreis wird die Umsetzung von Amin zu Carbaminsäurechlorid bei einem Druck von 10 bis 50 atü (ca. 11 bis 51 bar absolut) und einer Temperatur von 40 bis 120 °C durchgeführt. Der Reaktionskreis wird so betrieben, dass an einer Stelle der Ringleitung ein Aminstrom eingeführt wird, während an einer anderen, strömungstechnisch hinter dem Ort des Amineintrags liegenden Stelle des Reaktionskreises eine Mischung aus Frischphosgen und in der Kolonne zurückgewonnenem Phosgen eingeführt wird. Die so eingespeisten Edukte und aus ihnen gebildetes Carbaminsäurechlorid werden in der Ringleitung im Kreis geführt. An einer dritten, strömungstechnisch hinter dem Ort des Phosgeneintrags liegenden Stelle des Reaktionskreises wird je Zeiteinheit über einen in den Reaktionskreis integrierten Separator ein Teil des im Kreis geführten Reaktionsgemisches ausgetragen und der Kolonne zugeführt. Die Verwendung einer von einem einfachen Rohr zu unterscheidenden Mischeinrichtung, in welcher Amin, Phosgen und Carbaminsäurechlorid gleichzeitig vermischt werden, wird nicht offenbart. Phosgen wird bevorzugt in sehr großem Überschuss, beispielsweise 100 bis 500 %, eingesetzt, wahrscheinlich um Polymerisationsreaktionen zurückzudrängen. Die genannte Kolonne ist als Vorrichtung zur Spaltung von Carbaminsäurechlorid in Isocyanat und Chlorwasserstoff aufzufassen und wird bei einem Druck von mindestens 10 atü (ca. 11 bar absolut) betrieben. Unterhalb des Kolonnenkopfs wird in einem Seitenabzug Phosgen zurückgewonnen und über einen Vorratsbehälter in den Reaktionskreis zurückgeführt. Am Kolonnen-Kopf wird durch Wärmeüberträger bei 82 °C ein Chlorwasserstoff-Kondensat erhalten (rund 10 kg/h), das noch etwa 6 % Phosgen enthält (Beispiel 3). Die im Sumpf bei 142 °C erhaltene MMDI-Lösung wird danach zur Lösungsmittelrektifikationskolonne geführt; dieser Strom enthält neben MMDI (14,9 kg/h) auch noch beträchtliche Mengen an Phosgen (30 kg/h).

Ein Kreislaufverfahren mit anschließender Aufarbeitung für die Herstellung von Isocyanaten durch Phosgenierung, legt EP 0 716 079 B1 offen. Die Auslegung des Verfahrens lässt vorteilhaft den Verzicht einer Umwälzpumpe zu. Es werden Phosgenüberschüsse zwischen 110 und 300 % beansprucht. Die Edukte werden an separaten Stellen der Blasensäule zugeführt, wobei das Phosgen gasförmig und die Mischung bestehend aus MDA und Monochlorbenzol flüssig vorliegen. Durch die Chlorwasserstoff-Entwicklung wird die Reaktionslösung kontinuierlich umgewälzt. Das Verfahren wird in einem Temperaturbereich von 60 bis 100 °C sowie mit einem Druck von 0,5 und 5 bar betrieben.

Einen Kreislaufreaktor, der bei Drücken bis zu 14 kg/cm² betrieben wird, beschreibt DE-AS-1 037 444. Hierbei werden Amin, Phosgen sowie das inerte Lösungsmittel o-Dichlorbenzol an drei unterschiedlichen Stellen in den Mischkreis eingefahren, wobei eine Pumpe die Kreislauffahrweise erwirkt. Nach Vereinigung der genannten Stoffströme gelangt die Reaktionsmischung in einen Erhitzer und es werden Temperaturen oberhalb 110 °C erreicht. Über ein Drosselventil wird entspannt und das Gemisch gelangt sodann in einen Sammelbehälter, der unter Atmosphärendruck betrieben wird. Die gasförmigen Stoffe werden am Kopf des Sammelbehälters entnommen und über einen Kondensator als eine Mischung bestehend aus Chlorwasserstoff und Phosgen gewonnen. Am Sumpf des genannten Behälters wird ein Teil der Isocyanatlösung zurückgeführt und der andere einer weiteren Trennoperation unterzogen. Phosgenüberschüsse von mindestens 96 % sind erforderlich, um im beschriebenen Reaktor bei geringem Überdruck von 0,07 kg/cm² eine Ausbeute an 4,4'-Diphenylmethandiisocyanat von 90,5 % zu erhalten.

Ein zweistufiges Herstellverfahren für Isocyanate wird in DE 32 12 510 C3 beschrieben, wobei eine Mischung bestehend aus Isocyanaten und dem entsprechenden Carbaminsäurechlorid unter Anwesenheit von Phosgen und inertem Lösungsmittel zurückgeführt wird. Die erste Reaktionsstufe wird in einem tankförmigen Gefäß oder in einer röhrenförmigen Umlaufleitung bei Temperaturen von 60 bis 100 °C sowie einem absoluten Druck von 4 bis 8 bar durchgeführt. Zur Vervollständigung des Umsatzes an intermediär gebildeten Carbaminsäurechlorid wird die Reaktionsmischung bei gleichem Druck unter erhöhter Temperatur von 120 bis 160 °C in eine zweite Stufe geführt, um eine Isocyanatkonzentration von 10 bis 25 % anzusteuern. Durch die gewählten Reaktionsbedingungen kann die beschriebene Anlage vorteilhafterweise in rostfreiem Stahl anstelle höherwertiger Werkstoffe ausgekleidet sein. Die Reaktionsmischung liegt im beschriebenen Verfahren als Aufschlämmung vor (= Suspension). Der beschriebene Phosgenüberschuss beträgt wenigstens 100 %, und es wird Chlorwasserstoff bei maximal 10,8 bar abgeführt.

Zur Vereinigung der Edukte mit rückgeführtem Reaktionsgemisch beschreibt DE 26 24 285 C2 die Nutzung einer Treibstrahldüse, die vorteilhaft eine intensive Mischung in kurzer Zeit ermöglicht. Durch den angewandten Druckbereich von 1 bis 10 bar konnten neben Roh-MDI auch Toluylendiisocyanat-Isomerengemisch, 1,5-Naphthylendiisocyanat und 1,4-Phenylendiisocyanat in hohen Ausbeuten bei Verweilzeiten von 10 bis 180 Minuten erhalten werden. Eine Druckerhöhung zur Ausbeutesteigerung konnte dabei nicht festgestellt werden, der Phosgenüberschuss betrug in Bezug auf MDA als Aminkomponente größer als 100 %.

In DE-OS-2 252 068 wird ein Verfahren zur Phosgenierung von Aminen zu Isocyanaten beschrieben, das in einer Vorrichtung lösungsmittelfrei unter Rückführung der hergestellten Isocyanate bei einem Druck von bis zu 100 atm und einer Temperatur von bis zu 240 °C betrieben wird. Dabei wird zunächst verflüssigtes Amin mit einem Gemisch bestehend aus Phosgen und rückgeführtem Isocyanat adiabat bei 100 atm und 150 °C in einem Röhrenreaktor zur Reaktion gebracht, wobei eine Temperatur von 240 °C erreicht wird. Im Anschluss daran wird das Reaktionsgemisch isentropisch auf 20 atm entspannt und frisches Phosgen-/Isocyanatgemisch zugeführt. Neben Isocyanaten wird durch destillative Aufarbeitung der gasförmigen Komponenten Chlorwasserstoff mit einem Druck von 3 atm erhalten und Phosgen wird flüssig dem Prozess zurückgeführt.

Ein zweistufiges Verfahren zur Produktion von Isocyanat legt DE-OS-2 058 032 offen, wobei während der Heißphosgenierung die Temperatur allmählich angehoben wird. Dabei kann ein Phosgenüberschuss von beispielsweise 8 % angewendet werden, um Isocyanate in Ausbeuten zwischen 90 und 95 % zu erhalten. Konkret gezeigt wird dies nur für die Phosgenierung des monofunktionellen Amins Anilin. Für heutige Anforderungen sind diese Ausbeuten zu gering; außerdem ist fraglich, ob sich die in dieser Schrift offenbarten Erkenntnisse ohne Weiteres auf die Phosgenierung von Polyaminen, bei der die Gefahr von Polymerisationsreaktionen besteht, übertragen lassen. Besonders die Bildung von Polyharnstoffen durch Polyaddition von Polyaminen mit Polyisocyanaten sei hierbei erwähnt. Die beschriebene Anlage beinhaltet als wesentlichen Teil ein mechanisch durchmischtes, waagrechtes Rohr, worin die Temperatur allmählich von 30 auf 150 °C erhöht wird, und das den Kaltphosgenierungsteil mit dem Entgasungsrohr verbindet.

Eine Kreislaufvorrichtung zur Herstellung von Isocyanaten wird in EP-A-1 867 632 beschrieben, welche im Wesentlichen aus einer Zirkulationsleitung, einer Polyamin- Carbonylchlorid-Kontakteinheit und einer Mischeinheit durch Scherwirkung besteht. Zwischen Kontakt- und Mischeinheit wird ein Abstand von 1000 mm und weniger beansprucht. Der Vorteil der beschriebenen Vorrichtung liegt darin, dass die Bildung von Harnstoffen als Nebenkomponenten durch die effektivere Vermischung beider Reaktanden vermindert wird. Es werden Phosgenüberschüsse von 0 % bis 5900 % (2 ≤ n(COCl2)/n(Polyamin) ≤ 60) genannt. Der Erfindungsgegenstand ermöglicht es laut den Angaben der Schrift, die Bildung von harnstoffartigen Nebenkomponenten zu unterdrücken, was ausbeuteerhöhend in Bezug auf Polyisocyanat wirkt. Beispiele, die den beanspruchten Vorteil zeigen könnten, sind in der Anmeldung allerdings nicht beschrieben. Insbesondere gibt es keinen Nachweis, dass auch bei geringen oder gar keinen Phosgenüberschüssen großtechnisch akzeptable Ausbeuten erzielt werden. Zwischen Kontakt- und Mischeinheit wird ein Abstand von 1000 mm oder weniger beansprucht, die Eduktströme werden dabei mit einer Lineargeschwindigkeit von 0,5 bis 10 m/s in die Reaktionslösung (0,3 bis 5 m/s) über Rohre eingebracht. Form und Konstruktion sind in der Anmeldung als Zeichnung gezeigt. Ferner wird in der Beschreibung der Anmeldung angegeben, dass durch Reaktion von HCl mit Polyamin zu Polyaminhydrochlorid die Bildung von Carbamoylchloriden und Polyisocyanaten minimiert wird. In der Zirkulationsleitung findet aufgrund der Generierung eines laminaren Strömungsprofils keine Reaktion statt.

Ein weiteres Verfahren zur Herstellung von Isocyanaten wird in EP 0 150 435 B1 offengelegt. Vor Rückführung der sich im Kreislauf befindlichen Reaktionsmischung wird dabei Chlorwasserstoff abgetrennt, um Konzentrationen unterhalb 0,5 Gew.-% vor der Aminzugabe zu erhalten. Intermediäre Salzbildung und Nebenproduktbildung wird bei diesem Verfahren vorteilhafterweise durch die Abtrennung des Chlorwasserstoffs unterdrückt und als Folge die Isocyanatkonzentration im Reaktor erhöht. Das Molverhältnis zwischen Phosgen und Amingruppen beträgt 12 : 1 bis 200 : 1. Das abgetrennte Chlorwasserstoffgas wird unter hohem Druck gewonnen. Über eine Treibstrahldüse werden die Reaktanden mit dem Rückführstrom, bei dem es sich hauptsächlich um in Monochlorbenzol gelöstes Isocyanat handelt, gemischt, wobei im Mischkreis eine Temperatur von 130 °C und ein Druck von 14,5 bar herrschen. Zur Trennung von Chlorwasserstoff und Phosgen wird eine Kolonne verwendet, wobei das im Sumpf erhaltene Phosgen dem Prozess zurückgeführt wird.

Den vorstehend beschriebenen Verfahren ist gemeinsam, dass sie in der Praxis, um sehr gute Ausbeuten an Polyisocyanaten zu erzielen, sehr große Phosgenüberschüsse benötigen. Insbesondere im Fall der Herstellung von MMDI und PMDI ist nach dem Stand der Technik ein hoher Phosgenüberschuss unabdingbar, um akzeptable Ausbeuten (die in der großtechnischen Produktion aus Wirtschaftlichkeitsgründen > 99 % sein sollten) zu erreichen. Hohe Phosgenüberschüsse sind jedoch sowohl aus Wirtschaftlichkeits- als auch aus Sicherheitsgründen wenig erstrebenswert. Es bestand daher ein Bedarf an einem Verfahren zur Produktion von Polyisocyanaten, bei dem der Phosgenüberschuss gering gehalten werden kann, ohne dass dies anderweitige Nachteile (wie verringerte Ausbeute oder erhöhte Polymerisationsneigung mit einhergehender Gefahr der Bildung von Ablagerungen) mit sich bringt.

Dem vorstehend Gesagten Rechnung tragend ist ein Gegenstand der vorliegenden Erfindung ein kontinuierliches Verfahren zur Herstellung eines Polyisocyanats in der Flüssigphase durch Umsetzung des korrespondierenden Polyamins mit Phosgen, wobei als Zwischenstufen das entsprechende Carbaminsäurechlorid und das entsprechende Aminhydrochlorid auftreten, bei dem
(i) ein Phosgen-haltiger Strom (1), ein Polyamin-haltiger Strom (2) und ein Carbaminsäurechlorid- und Aminhydrochlorid-haltiger Strom (3) in einer Mischeinrichtung (1000) vermischt werden, wobei Phosgen in einem stöchiometrischen Überschuss von > 0 % bis 50 % der Theorie, bevorzugt von > 0 % bis 20 % der Theorie, besonders bevorzugt von > 0 % bis 15 % der Theorie, ganz besonders bevorzugt von > 0 % bis 10 % der Theorie, bezogen auf die Amingruppen des in Strom (2) enthaltenen Polyamins, eingesetzt wird,
(ii) der in Schritt (i) erhaltene Mischstrom (4) durch einen Reaktor (2000), in dem ein absoluter Druck von 20 bar bis 60 bar und eine Temperatur von 80 °C bis 200 °C, bevorzugt ein absoluter Druck von 20 bar bis 55 bar und eine Temperatur von 80°C bis 170°C, besonders bevorzugt ein absoluter Druck von 20 bar bis 50 bar und eine Temperatur von 80°C bis 150°C, herrschen, geführt wird, sodass das eingesetzte Polyamin weitgehend bis vollständig, bevorzugt vollständig, umgesetzt wird, wobei ein Carbaminsäurechlorid- und Aminhydrochlorid-haltiger Strom (5) gebildet wird,
(iii) optional ein gasförmiger Purge-Strom aus dem in Schritt (ii) gebildeten Carbaminsäurechlorid- und Aminhydrochlorid-haltigen Strom (5) ausgeschleust wird, wobei ein an gasförmigen Anteilen abgereicherter Carbaminsäurechlorid- und Aminhydrochlorid-haltiger Strom (6) erhalten wird,
(iv) der in Schritt (ii) erhaltene Strom (5) oder der in Schritt (iii) erhaltene Strom (6) in zwei Teilströme (7, 8) getrennt wird, wobei
   (a) der Teilstrom (7) als Carbaminsäurechlorid- und Aminhydrochlorid-haltiger Strom (3) in Schritt (i) eingesetzt wird und
   (b) der Teilstrom (8) zum gewünschten Polyisocyanat umgesetzt wird.

Im Rahmen der vorliegenden Erfindung werden die Begriffe *Carbaminsäurechlorid* und *Aminhydrochlorid* unabhängig davon verwendet, ob nur eine oder bereits alle Aminfunktionen -NH₂ des Ausgangs-Polyamins zu -NHCOCl bzw. -NH₃Cl umgesetzt sind.

*Polyamine* bzw. *Polyisocyanate* bezeichnen im Rahmen der vorliegenden Erfindung Stoffe, die mindestens zwei Amingruppen bzw. Isocyanatgruppen enthalten.

Unter einer *"Mischeinrichtung (1000)*" werden im Sinne dieser Erfindung solche Einrichtungen verstanden, die speziell für die Vermischung mehrerer Stoffströme konstruiert sind (siehe weiter unten für Details).

Das Wort *"ein"* ist im Rahmen der vorliegenden Erfindung im Zusammenhang mit zählbaren Größen nur dann als Zahlwort und nicht lediglich als unbestimmter Artikel zu verstehen, wenn dies ausdrücklich gesagt wird oder aus dem Zusammenhang eindeutig hervorgeht. Beispielsweise umfasst der Ausdruck *"ein Reaktor"* auch Ausführungsformen, in denen mehrere Reaktoren parallel oder in Serie geschaltet sind.

Nachstehend werden Ausführungsformen der Erfindung näher beschrieben. Dabei sind verschiedene Ausführungsformen beliebig miteinander kombinierbar, solange sich für den Fachmann aus dem Kontext nicht eindeutig das Gegenteil ergibt.

Die Erfindung befasst sich mit einem großtechnisch durchgeführten kontinuierlichen Prozess zur Polyisocyanat-Synthese. Zu Beginn eines solchen Prozesses, beispielsweise bei Wiederinbetriebnahme einer Anlage nach einem Produktionsstillstand, liegt naturgemäß noch kein Carbaminsäure- und Aminhydrochlorid-haltiger Strom (3) vor. Die Reaktion von Polyamin und Phosgen wird daher zunächst ohne Zugabe dieses Stroms angefahren. Sobald ein stabiler Betriebszustand des kontinuierlichen Produktionsprozesses erreicht ist, wird das Verfahren wie zuvor beschrieben betrieben, d. h. unter Zuführung des Stroms (3).

Das erfindungsgemäße Verfahren eignet sich z. B. für die Herstellung von Methylendiphenyldiisocyanat (MMDI) als reine Isomere oder als Isomerengemisch, Polymethylenpolyphenylpolyisocyanat (PMDI), Gemische aus Methylendiphenyldiisocyanat und Polymethylenpolyphenylpolyisocyanat, Toluylendiisocyanat (TDI) als reine Isomere oder Isomerengemisch, Isomere des Xylylendiisocyanats (XDI), Isomere des Diisocyanatobenzols, 2,6-Xylolisocyanat, 1,5-Naphthylendiisocyanat (1,5-NDI), Diisocyanate auf Basis von aliphatischen oder cycloaliphatischen Kohlenwasserstoffen mit 2 bis 18 Kohlenstoffatomen, wie z.B. 1,4-Butandiisocyanat, 1,5-Pentandiisocyanat, 1,6-Hexandiisocyanat (HDI), 1,8-Octandiisocyanat, 1,9-Nonandiisocyanat, 1,10-Decandiisocyanat, 2,2-Dimethylpentan-1,5-diisocyanat, 2-Methyl-1,5-pentandiisocyanat (MPDI), 2,4,4(oder 2,2,4)-Trimethyl-1,6-hexandiisocyanat (TMDI), 1,3- und 1,4-Cyclohexandiisocyanat, 1-Isocyanato-3,3,5-trimethyl-5-isocyanatomethylcyclohexan (IPDI), 2,4-, oder 2,6-Diisocyanato-1-methylcyclohexan (H6-TDI), 1-Isocyanato-1-methyl-4(3)-isocyanatomethylcyclohexan (AMCI), 1,3(und/oder 1,4)-Bis(isocyanatomethyl)cyclohexan, Bis(isocyanatomethyl)norbornan (NBDI), 4,4'(und/oder 2,4')-Diisocyanatodicyclohexyl-methan, und (cyclo)aliphatische Triisocyanate mit bis zu 22 Kohlenstoffatomen, wie z. B. Triisocyanatocyclohexan, Tris(isocyanatomethyl)-cyclohexan, Triisocyanato-methylcyclohexan, 1,8-Diisocyanato-4-(isocyanatomethyl)octan, 1,6,11-Undecantriisocyanat, 1,7-Diisocyanato-4-(3-isocyanatopropyl)heptan, 1,6-Diisocyanato-3-(isocyanatomethyl)-hexan oder 1,3,5-Tris(isocyanatomethyl)-cyclohexan.

Die korrespondierenden Amine zu den obigen Polyisocyanaten sind aromatische Polyamine, wie z. B. Methylendiphenyldiamin (MMDA) als reine Isomere oder als Isomerengemisch, Polymethylenpolyphenylpolyamin (PMDA), Gemische aus Methylendiphenyldiamin und Polymethylenpolyphenylpolyamin, Toluylendiamin (TDA) als reine Isomere oder Isomerengemisch, Isomere des Xylylendiamins (XDA), Isomere des Diaminobenzols, 2,6-Xylidin, 1,5-Naphthylendiamin (1,5-NDA), Polyamine auf Basis von aliphatischen oder cycloaliphatischen Kohlenwasserstoffen mit 2 bis 18 Kohlenstoffatomen, wie z.B. 1,4-Diaminobutan, 1,5-Diaminopentan, 1,6-Diaminohexan (HDA), 1,8-Diaminooctan, 1,9-Diaminononan, 1,10-Diaminodecan, 2,2-Dimethyl-1,5-diaminopentan, 2-Methyl-1,5-pentandiamin (MPDA), 2,4,4(oder -2,2,4)-Trimethyl-1,6-diaminohexan (TMDA), 1,3- und 1,4-Diaminocyclohexan, 1-Amino-3,3,5-trimethyl-5-aminomethylcyclohexan (IPDA), 2,4-, oder 2,6-Diamino-1-methylcyclohexan (H6-TDA), 1-Amino-1-methyl-4(3)-aminomethylcyclohexan (AMCA), 1,3(und/oder 1,4)-Bis(aminomethyl)cyclohexan, Bis(aminomethyl)norbornan (NBDA), 4,4'(und/oder 2,4')-Diaminodicyclohexyl-methan, (cyclo)aliphatische Polyamine mit bis zu 22 Kohlenstoffatomen, wie z. B. Triaminocyclohexan, Tris(aminomethyl)-cyclohexan, Triamino-methylcyclohexan, 1,8-Diamino-4-(aminomethyl)octan, 1,6,11-Undecantriamin, 1,7-Diamino-4-(3-aminopropyl)heptan, 1,6-Diamino-3-(aminomethyl)-hexan oder 1,3,5-Tris(aminomethyl)-cyclohexan.

Das erfindungsgemäße Verfahren eignet sich besonders für die Herstellung von Methylendiphenyldiisocyanat (MMDI) als reine Isomere oder als Isomerengemisch, Polymethylenpolyphenylpolyisocyanat (PMDI), Gemische aus Methylendiphenyldiisocyanat und Polymethylenpolyphenylpolyisocyanat, Toluylendiisocyanat (TDI) als reine Isomere oder Isomerengemisch, Isomere des Xylylendiisocyanats (XDI), Isomere des Diisocyanatobenzols, 2,6-Xylolisocyanat, 1,5-Naphthylendiisocyanat (1,5-NDI).

In einer besonders bevorzugten Ausführungsform ist ein Verfahren zur Herstellung von Diphenylmethandiisocyanat (MMDI) und/oder Polyphenylenpolymethylenpolyisocyanat (PMDI) Gegenstand der vorliegenden Erfindung. MMDI und PMDI werden im Rahmen dieser Erfindung auch, unabhängig von Polymerisationsgrad und Isomerenverteilung, summarisch als MDI bezeichnet; Analoges gilt für MDA.

Die Herstellung der korrespondierenden Polyamine ist aus dem Stand der Technik hinreichend bekannt und wird daher an dieser Stelle nicht im Detail ausgeführt. Im Fall des besonders bevorzugten Polyisocyanats MDI wird das korrespondierende Polyamin MDA durch säurekatalysierte Kondensation von Anilin und Formaldehyd erhalten. Dabei entsteht ein Gemisch aus der "Zweikernverbindung" MMDA (enthaltend zwei Benzolringe mit je einer Aminogruppe) und höheren Homologen PMDA ("Mehrkernverbindungen", enthaltend drei oder mehr Benzolringe mit je einer Aminogruppe). Bei den meisten großtechnisch üblichen Verfahren wird dieses Gemisch ohne vorherige Trennung in monomere und polymere Bestandteile phosgeniert. Eine Trennung in monomere Bestandteile und polymere Bestandteile findet daher meist erst auf der Stufe des Polyisocyanats statt. Dabei werden einerseits die Zweikernverbindung (MMDI) und andererseits ein Gemisch aus der Zweikernverbindung (MMDI) und den höheren Homologen (PMDI) erhalten.

In **Schritt (i)** des erfindungsgemäßen Verfahrens werden die Edukte Polyamin und Phosgen bevorzugt in einem unter den Reaktionsbedingungen inerten Lösungsmittel eingesetzt. Besonders bevorzugt ist ein Verfahren, bei dem
der Phosgen-haltige Strom (1) einen Massenanteil von 10 % bis 90 %, bevorzugt von 30 % bis 70 %, besonders bevorzugt von 55 % bis 65 %, an Phosgen und einen Massenanteil von 90 % bis 10 %, bevorzugt von 70 % bis 30 %, besonders bevorzugt von 45 % bis 35 %, eines inerten Lösungsmittels umfasst, jeweils bezogen auf die Gesamtmasse des Stroms (1),
der Polyamin-haltige Strom (2) einen Massenanteil von 10 % bis 50 %, bevorzugt von 30 % bis 45 %, besonders bevorzugt von 35 % bis 40 %, an Polyamin und einen Massenanteil von 90 % bis 50 %, bevorzugt von 65 % bis 70 %, besonders bevorzugt von 60 % bis 65 %, eines inerten Lösungsmittels umfasst, jeweils bezogen auf die Gesamtmasse des Stroms (2).

*Unter den Reaktionsbedingungen inert* bedeutet dabei, dass das Lösungsmittel nicht in signifikantem Umfang mit den Edukten, Zwischenprodukten und Endprodukten der Reaktion reagiert. Daher wird bevorzugt das inerte Lösungsmittel für Strom (1) und Strom (2) unabhängig voneinander ausgewählt aus Chlorbenzol, Dichlorbenzol, Toluol, Dioxan, Dimethylsulfoxid oder einer Mischung aus zwei oder mehr der vorgenannten Lösungsmittel. Besonders bevorzugt für beide Ströme (1) und (2) sind Chlorbenzol und Dichlorbenzol, ganz besonders bevorzugt ist Chlorbenzol. Im Fall des Dichlorbenzols ist das ortho-Isomer (ortho-Dichlorbenzol) besonders bevorzugt.

Die in Schritt (i) eingesetzte Mischeinrichtung muss die gleichzeitige Vermischung von drei Prozessströmen (Carbaminsäurechlorid-/Aminhydrochlorid-, Polyamin- und Phosgen-haltige Ströme (3), (2) bzw. (1)) ermöglichen, wobei die Mischeinrichtung so ausgestaltet sein sollte, dass zeitweise (z. B. beim Wiederanfahren einer Produktionsanlage nach einem Stillstand) auch nur zwei Ströme miteinander vermischt werden können (Polyamin- und Phosgen-haltiger Strom (2) und (1)).

Die zu mischenden Prozessströme (1), (2) und (3) sollen bevorzugt rasch ohne nennenswerte Verweilzeitstrecke innig miteinander vermischt werden, so dass eine hohe Produktausbeute ermöglicht wird. Die Mischung der Komponenten in einem einfachen Rohr erfüllt diese Anforderungen nicht. Diese Voraussetzung wird von vielen in der Literatur beschriebenen Mischaggregaten erfüllt. Hierzu gehören zum einen bestimmte Typen von *statischen Mischern,* und hier insbesondere Düsen, wie beispielsweise Ringschlitzdüsen (DE-A-1792660), Ringlochdüsen (DE-C1-3744001), Glattstrahlmischdüsen (EP-A1-0 065 727), Fächerstrahldüsen (DE-A1-2950216), Winkelstrahlkammerdüsen (DD-A7-300.168), Dreistromdüsen (DD-A1-132340), Gegenstrommischkammern (DE-B-1146872), Staudüsen (FR-E-69428) und Venturimischdüsen (DE-B-1175666). Zum anderen wurden *dynamische Mischer* beschrieben, welche diese Voraussetzung erfüllen. Hierbei handelt es sich u. a. um Rotor-Stator- oder turbinenartige Systeme, bei denen die Reaktanden im Gleichstrom in die Mischeinheit eingeleitet werden. Als Beispiel kann EP-A-2 077 150 genannt werden. Dynamische Mischer werden im erfindungsgemäßen Verfahren bevorzugt eingesetzt, weil sich die Primärvermischung sowohl der Edukte (Phosgen- und Polyamin-haltige Ströme (1) und (2)) als auch des als Verdünnungsmittel wirkendem Carbaminsäurechlorid- und ggf. Aminhydrochlorid-haltigen Rückführstroms (3) als besonders effektiv erweist. Für die Herstellung von Polyisocyanaten mittels Phosgenierung aus den entsprechenden Polyaminen, die als schnell einzusetzende Reaktion aufzufassen ist, erwiesen sich diejenigen Mischaggregate als besonders vorteilhaft, welche einen zur Vermischung benötigten hohen Energieeintrag selbst bei Unterschieden im Viskositätsverhältnis η' von Amin- (2) zu Phosgenlösung (1), η' = η(2)/η(1), von sowohl kleiner als 0,5 als auch größer 2 gewährleisten (EP 2 077 150 A1).

Bei der Umsetzung von Polyaminen mit Phosgen wird durch die - unvermeidliche - Bildung von Aminhydrochloridpartikeln als Nebenprodukt neben der Selektivität zum gewünschten Carbaminsäurechlorid auch die Raum-Zeit-Ausbeute des Prozesses vermindert. Nach dem Stand der Technik ist es üblich, zur Steigerung der Ausbeute die *in-situ* gebildeten Aminhydrochloridpartikel bei erhöhten Temperaturen mit einem sehr großen Überschuss an Phosgen umzusetzen. Die Geschwindigkeit der Umsetzung ist wie in der Literatur beschrieben dabei primär von der mittleren Partikelgröße bestimmt, die sich, in Abhängigkeit der Mischgüte zu Beginn der Reaktion, vom Nano- über Mikro- bis hin zum Millimeterbereich erstrecken kann. Mit höherem Grad an Mischgüte wird eine feinteiligere Dispersion erzielt. Rohre für Amin- oder Phosgenlösung, die beide Edukte in den Reaktionskreis eintragen und wie sie in der Offenlegungsschrift DE-OS-1 593 412 beschrieben sind, erfüllen die Mischaufgabe nur unzureichend, mit dem damit verbundenen Nachteil der erforderlichen Anwendung eines hohen Phosgenüberschusses zur Vervollständigung der Reaktion des Amins zum gewünschten Carbaminsäurechlorid.

Es wurde nun gefunden, dass bei Verwendung von Mischaggregaten statischen oder dynamischen Typs die kontinuierliche Phosgenierung von Polyaminen unter Rückführung eines Carbaminsäure- und Aminhydrochlorid-haltigen Stroms bei einem deutlich niedrigeren Phosgenüberschuss als in der Literatur beschrieben durchgeführt werden kann.

Als Reaktor in **Schritt (ii)** kann grundsätzlich jeder aus dem Stand der Technik bekannte Phosgenierreaktor eingesetzt werden. Bevorzugt werden senkrecht stehende und von unten durchströmte Rohrreaktoren verwendet. Zur Einengung der Verweilzeit kann der Rohrreaktor durch geeignete, dem Fachmann bekannte Einbauten segmentiert sein.

Im Reaktor (2000) wird das Polyamin zu Carbaminsäurechlorid- und Aminhydrochlorid umgesetzt. Die Bildung von Polyisocyanat auf dieser Stufe kann natürlich nicht völlig ausgeschlossen werden, erfolgt aber, als Folge des erfindungsgemäß anzuwendenden hohen Drucks in Schritt (ii), im Allgemeinen nur in untergeordnetem Ausmaß. Durch geeignete Wahl der Reaktionsbedingungen (siehe weiter unten für Details) ist es möglich, das Verhältnis von Carbaminsäurechlorid zu Aminhydrochlorid zu Gunsten des Ersteren zu verschieben, was wünschenswert ist, weil die Phosgenierung von Aminhydrochlorid erfahrungsgemäß eine langsame Reaktion ist. Völlig vermeiden lässt sich die Anwesenheit von Aminhydrochlorid in Strom (5) jedoch nicht.

In einer Ausführungsform des erfindungsgemäßen Verfahrens wird der Reaktor (2000) adiabat, d. h. ohne gezielte Wärmezu- oder -abfuhr, betrieben. In einem solchen Verfahren spiegelt sich die Reaktionsenthalpie - unter Vernachlässigung unvermeidbarer Wärmeverluste - quantitativ in der Temperaturdifferenz zwischen Ausgangs- und Eingangsstrom wider. Zur Vermeidung von Wärmeverlusten wird der Reaktor bevorzugt isoliert. In der Druckschrift EP 1 616 857 A1 ist die adiabate Betriebsweise bei der Polyaminphosgenierung näher beschrieben, insbesondere in den Absätzen [0014] bis [0018].

In einer anderen Ausführungsform des erfindungsgemäßen Verfahrens wird der Reaktor (2000) isotherm, d. h. unter Zufuhr von Wärme über einen thermostatisierbaren Reaktor durch ein geeignetes Wärmeträgermedium (z. B. Wärmeträgeröl, Salzschmelze), betrieben. Beispielhaft seien die Schriften DE 1768439 A1, insbesondere Absatz [0003] auf Seite 8 und EP 1 616 857 B1, insbesondere die Absätze [0021] bis [0022], für die Phosgenierung von Polyaminen unter isothermer Betriebsweise genannt.

Unabhängig davon, ob der Reaktor (2000) adiabat oder isotherm betrieben wird, wird die Reaktion bevorzugt so geführt, dass das Verhältnis n' der Stoffmengen von Carbaminsäurechlorid (CSC) und Aminhydrochlorid (AHC) einerseits zu Polyisocyanat (PIC) andererseits, n' = [n(CSC) + n(AHC)] / n(PIC), in dem den Reaktor 2000 verlassenden Produkt Strom (5) 2 : 1 bis 100 : 1, bevorzugt 10 : 1 bis 80 : 1, besonders bevorzugt 30 : 1 bis 60 : 1, beträgt. Diese Stoffmengenverhältnisse werden bevorzugt durch geeignete Wahl des in (2000) vorliegenden absoluten Drucks eingestellt und können vom Fachmann bei bekannten Randbedingungen berechnet werden. Ein höherer Druck in (2000) führt dazu, dass der Anteil gelösten Chlorwasserstoffes zunimmt, sodass das Gleichgewicht zwischen Polyisocyanat und Carbaminsäurechlorid bzw. Aminhydrochlorid auf die Seite des Carbaminsäurechlorids bzw. Aminhydrochlorids verschoben wird. Eine Verallgemeinerung der Abhängigkeit des Stoffmengenverhältnisses n' vom absoluten Druck in (2000) ist, aufgrund der vielfältigen Abhängigkeiten von den übrigen Randbedingungen, schwierig. Der Fachmann ist jedoch in der Lage, abhängig vom eingesetzten Polyamin, der Temperatur und den anderen in einer gegebenen Produktionsanlage vorherrschenden Randbedingungen durch einfache Vorversuche und/oder rechnerische Simulationen den Zusammenhang zwischen absolutem Druck in (2000) und dem Stoffmengenverhältnis Carbaminsäurechlorid zu Polyisocyanat in dem den Reaktor (2000) verlassenden Produkt Strom (5) zu quantifizieren.

Aus dem in Schritt (ii) im Reaktor (2000) gebildeten Carbaminsäure- und Aminhydrochlorid-haltigen Strom (5) wird optional in **Schritt (iii)** ein gasförmiger Purge-Strom (9) ausgeschleust, wobei ein an gasförmigen Anteilen abgereicherter Carbaminsäurechlorid- und Aminhydrochlorid-haltiger Strom (6) erhalten wird. Dies geschieht in aus dem Stand der Technik bekannten Apparaturen (3000), beispielsweise Gas-Flüssig-Abscheidern oder Kolonnen. Die zur Entnahme des gasförmigen Purge-Stromes verwendete Vorrichtung (3000) kann auch in den Reaktor (2000) integriert sein. Bevorzugt entspricht die optional in Schritt (iii) ausgeschleuste Menge des gasförmigen Purge-Stromes > 0 bis < 1,0 Massen-% des Carbaminsäure- und Aminhydrochlorid-haltigen Strom (5), bezogen auf die Gesamtmasse dieses Stroms.

In **Schritt (iv)** wird entweder der in Schritt (ii) erhaltene Strom (5) (in der Ausführungsform ohne Schritt (iii)) oder der in Schritt (iii) erhaltene Strom (6) (in der Ausführungsform mit Schritt (iii)) in zwei Teilströme (7, 8) getrennt. In einer bevorzugten Ausführungsform umfasst Strom (7) 5,0 Massen % bis 95 Massen % und Strom (8) von 5,0 Massen % bis 95 Massen % des in Schritt (iii) erhaltenen Stroms (5) [in der Ausführungsform ohne Schritt (iii)] oder des in Schritt (iii) erhaltenen Stroms (6) [in der Ausführungsform mit Schritt (iii)], jeweils bezogen auf die Gesamtmasse des Stroms (5) bzw. (6).

Der Teilstrom (7) wird in die Reaktion zurückgeführt (Reaktionskreislauf), und zwar als Strom (3) in Schritt (i) (**Schritt** (**iv)** (**a**)).

Der Teilstrom (8) wird, wie nachstehend näher erläutert, zum gewünschten Polyisocyanat (10) aufgearbeitet (**Schritt** (**iv**) (**b**)).

Insbesondere dann, wenn der Strom (8) größere Mengen an Aminhydrochlorid enthält, wird der Strom (8) bevorzugt zunächst durch einen weiteren Reaktor (5100), der isotherm betrieben wird, geführt. Größere Mengen an Aminhydrochlorid machen sich durch eine Trübung der Reaktionslösung bemerkbar. Diese lässt sich im einfachsten Fall durch optische Beobachtung mittels eines Schauglases feststellen. Im Reaktor (5100) wird Aminhydrochlorid phosgeniert. Der dem Reaktor (5100) entnommene Produktstrom wird dann durch eine Kaskade an Abscheidern mit sukzessiv abnehmendem Druck geführt. Dabei wird Carbaminsäurechlorid durch Entspannung zu Polyisocyanat umgesetzt und überschüssiges Phosgen mit der Gasphase abgetrennt. Um eine deutliche Reduktion der Betriebstemperatur der einzelnen Apparate bei Entspannung zu vermeiden, wird der Druck- und Temperaturbereich in der Weise eingestellt, dass während des Betriebs nicht mit Feststoffbildung zu rechnen ist. Beispielsweise kann der absolute Druck zweistufig ausgehend von den im Reaktor (5100) herrschenden Druck- und Temperaturverhältnissen über eine Zwischenstufe bei 12 bar/100 °C auf Umgebungsdruck und -temperatur reduziert werden.

Abhängig von der genauen Ausgestaltung des Reaktionskreislaufs, insbesondere des Betriebs des Reaktors (2000), kann der Reaktor (5100) entfallen und der Strom (8) direkt durch eine Kaskade an Abscheidern mit sukzessiv abnehmendem Druck geführt werden. Der Verzicht auf Reaktor (5100) wird durch nahezu vollständige Umsetzung von Aminhydrochlorid im Reaktor (2000) ermöglicht, was insbesondere dann realisierbar ist, wenn (2000) isotherm und nicht adiabat betrieben wird. Auch die mittlere Verweilzeit des ersten Reaktors beeinflusst den Aminhydrochloridgehalt in Strom (8). Bei erhöhtem Reaktorvolumen für (2000) und daraus resultierender Erhöhung der mittleren Verweilzeit wird die Umsetzung des Aminhydrochlorids vollständiger erfolgen.

Die Zugabe weiteren Phosgens in Schritt (v) (b) ist, unabhängig davon, ob der Reaktor (5100) eingesetzt wird oder nicht, im erfindungsgemäßen Verfahren nicht erforderlich. Da im Reaktor (2000) nur geringe Phosgenüberschüsse eingesetzt werden, ergibt sich damit insgesamt ein überaus geringer Phosgenbedarf, verglichen mit üblichen großtechnischen Verfahren. Dies ist unter Sicherheitsaspekten, aber auch aus wirtschaftlichen Gründen, höchst wünschenswert. Dabei werden mit dem erfindungsgemäßen Verfahren trotz der geringen Phosgenüberschüsse hervorragende Ausbeuten erzielt. Ein weiterer Vorteil des erfindungsgemäßen Verfahrens liegt darin, dass infolge der geringeren Phosgenüberschüsse die Aufarbeitung vereinfacht wird, wie nachfolgend beschrieben wird.

Der so erhaltene Roh-Polyisocyanat-Strom (10) kann grundsätzlich nach allen aus dem Stand der Technik bekannten Verfahren zum Rein-Polyisocyanat aufgearbeitet werden. Dies wird beispielsweise durch eine Kaskade an Rektifikationskolonnen realisiert. Im erfindungsgemäßen Verfahren wird diese Rektifikationssequenz verglichen mit dem Stand der Technik jedoch dahingehend vereinfacht, dass infolge eines geringen Phosgenüberschusses eine deutlich geringere Menge an Phosgen, welches aus wirtschaftlichen Gründen dem Prozess wieder zur Verfügung gestellt werden soll, zurückgeführt werden muss. Prozessstufen zur Verflüssigung und/oder zur Lösung des überschüssigen Phosgens in einem Lösungsmittel (Phosgenabsorption) können somit erheblich verkleinert werden oder sogar ganz entfallen, was den Kapitalaufwand für den Bau einer entsprechenden Anlage spürbar reduziert. Im Idealfall ist der Phosgenüberschuss so gering, dass die geringen Mengen anfallenden nicht umgesetzten Phosgens nicht wie in den Verfahren des Standes der Technik üblich auf aufwändige Weise, beispielsweise über Phosgenabsorption mit Chlorbenzol als Absorbens bei Temperaturen unterhalb von 0 °C, zurückgewonnen werden müssen. Vielmehr kann diese Rückgewinnung des Phosgens im erfindungsgemäßen Verfahren über eine einfache Kondensation mit indirekter Kühlung erfolgen. Zurückgewonnenes Phosgen kann als Bestandteil von Strom (1) in Schritt (i) eingesetzt oder einem anderen Verwendungszweck für Phosgen zugeführt werden. In einer alternativen Ausführungsform wird das restliche Phosgen vernichtet, da die anfallenden Mengen im günstigsten Fall so gering sind, dass eine stoffliche Wiederverwertung wirtschaftlich nicht zwingend erforderlich ist.

Ausführungsformen der vorliegenden Erfindung werden anhand der beigefügten Zeichnungen näher erläutert.

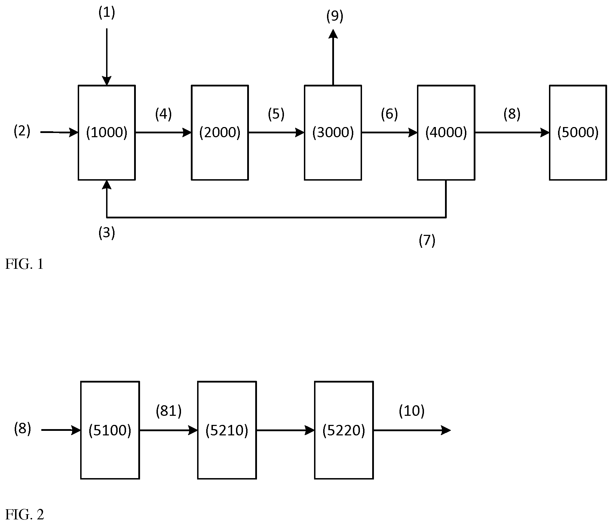

FIG. 1 zeigt schematisch und stark vereinfacht eine Ausführungsform des erfindungsgemäßen Verfahrens:
   Einer Mischeinrichtung (1000) werden ein Phosgenstrom (1), ein Polyaminstrom (2) und ein Carbaminsäure- und Aminhydrochlorid-haltiger Strom (3) zugeführt und dort innig vermischt (Schritt (i) des erfindungsgemäßen Verfahrens), wobei alle drei Ströme ein inertes Lösungsmittel enthalten. Der resultierende Mischstrom (4) wird durch den Reaktor (2000) geführt, wobei das Polyamin umgesetzt wird, sodass ein Carbaminsäurechlorid- und Aminhydrochlorid-haltiger Strom (5) gebildet wird (Schritt (ii) des erfindungsgemäßen Verfahrens). In einer nachgeschalteten Vorrichtung (3000) wird ein gasförmiger, überwiegend Chlorwasserstoff umfassender, Purge-Strom (9) entnommen (Schritt (iii) des erfindungsgemäßen Verfahrens). Der verbleibende flüssige Strom (6) wird in einer Vorrichtung (4000) in zwei Teilströme (7) und (8) aufgeteilt (Schritt (iv) des erfindungsgemäßen Verfahrens). Strom (7) wird als Carbaminsäurechlorid- und Aminhydrochlorid-haltiger Strom (3) in die Mischeinrichtung (1000) zurückgeführt (Schritt (iv) (a) des erfindungsgemäßen Verfahrens), während Strom (8) in (5000) zum Polyisocyanat (10) aufgearbeitet wird (Schritt (iv) (b) des erfindungsgemäßen Verfahrens).
FIG. 2 zeigt eine mögliche Ausgestaltung der in (5000) durchgeführten Umsetzung des in Strom (8) enthaltenen Carbaminsäurechlorids und Aminhydrochlorids zum Polyisocyanat in Strom (10):
   Strom (8) wird zunächst einem isotherm betriebenen Reaktor (5100) zugeführt (Schritt (iv) (b) (1)), und der den Reaktor verlassende Strom (81) wird durch eine Kaskade (5200) an Abscheidern (5210, 5220) mit sukzessiv abnehmendem Druck geführt (Schritt (iv) (b) (2)). Die in der Abbildung gezeigte Ausführungsform mit zwei Abscheidern (5210, 5220) ist als exemplarisch zu sehen. Nach Durchlaufen des letzten Abscheiders (5220) wird ein Polyisocanatstrom (10) erhalten, der keine nennenswerten Anteile an Aminhydrochlorid mehr enthält und gemäß einem Verfahren des Standes der Technik zu Rein-Polyisocyanat aufgearbeitet wird, wobei das eingesetzte Lösungsmittel zurückgewonnen wird (nicht gezeigt in FIG. 2). Im Polyisocanatstrom (10) können ggf. noch geringe Mengen an CSC enthalten sein, die in der weiteren Aufarbeitung zum Polyisocyanat gespaltet werden.

### Beispiele

Die im Folgenden dargestellten Beispiele basieren auf einer Prozesssimulation des stationären Betriebszustands, wie sie z. B. in U. Plöcker, R. Janowsky, H. Briesen, W. Marquardt, "Prozessanalyse und -synthese: Modellierung, Simulation und Optimierung", Kapitel 9 "Stationäre und dynamische Prozesssimulation" in "Chemische Technik", Winnacker, Küchler (Hrsg.), Band 2, 5. Aufl., Wiley-VCH-Verlag, Weinheim, 2004, S. 161ff. in ihren wesentlichen Zügen beschrieben wird. Zur Lösung des stationären Betriebszustands lagen dem Modell einerseits die Phasengleichgewichte der einzelnen Komponenten und zum anderen die Reaktionskinetik der Umsetzung von MDA mit Phosgen unter Einhaltung der Energie- und Massenbilanz zu Grunde.

### Beispiel 1 (erfindungsgemäß, adiabate Betriebsweise in Reaktor (2000), isotherm betriebener Nachreaktor (5100)):

In einer Mischeinrichtung wurden 5 kg/h 4,4'-Diaminodiphenylmethan (MMDA) als 41,7-massenprozentige Lösung in ortho-Dichlorbenzol (Strom (2)), 5,3 kg/h Phosgen als 58-massenprozentige Lösung in ortho-Dichlorbenzol (Strom (1)) mit einem rückgeführtem Carbaminsäure-haltigen Strom von 37,6 kg/h (Strom (3)) vereinigt, wobei die Konzentration an Carbaminsäurechlorid etwa 25 % in ortho-Dichlorbenzol betrug. Es wurde ein stoffmengenbezogener Phosgenüberschuss auf das eingesetzte Amin von 5 % der Theorie dabei eingestellt **(Schritt (i)** des erfindungsgemäßen Verfahrens). Die Temperatur am Austritt der Mischeinrichtung betrug 131 °C, und es herrschte ein Druck von 40 bar über Atmosphärendruck. Das Reaktionsgemisch (Strom (4)) gelang anschließend in einen turmförmigen Reaktor ((2000), **Schritt (ii)** des erfindungsgemäßen Verfahrens), dem zur Vermeidung von Thermalexpansionen stündlich 600 g Chlorwasserstoff als gasförmige Komponente am Kopf entnommen wurde **(Schritt (iii)** des erfindungsgemäßen Verfahrens). Der verbliebene flüssige Stoffstrom (Strom (6)) wurde anschließend mit einem massenbezogenen Verhältnis von 1,85 : 1 in zwei Ströme bei einer Temperatur von 105 °C aufgetrennt **(Schritt (iv)** des erfindungsgemäßen Verfahrens), wobei 20,2 kg/h (Strom (8)) des Stromes in einen Nachreaktor (5100) geführt wurden **(Schritt (iv) (b) (1)** des erfindungsgemäßen Verfahrens). Der andere Teil (Strom 7)) des Stromes wurde wie oben beschrieben in die Mischeinrichtung zurückgeführt **(Schritt (iv) (a)** des erfindungsgemäßen Verfahrens). Im Nachreaktor wurde der Stoffstrom (8) auf 140 °C aufgeheizt, und der Druck betrug 40 bar über Atmosphärendruck. Anschließend wurde über mehrere Separatoren der Druck von 40 bar über Atmosphärendruck auf atmosphärischen Umgebungsdruck reduziert **(Schritt (iv) (b)(2)** des erfindungsgemäßen Verfahrens). Die erhaltene flüssige Roh-MMDI-Lösung, die kein MMDA mehr enthielt und einen Gehalt an Harnstoffgruppierungen enthaltenen Nebenkomponenten von 0,5 Massen-%, bezogen auf die Masse des in der Roh-MMDI-Lösung enthaltenen MMDI, aufwies, wurde destillativ gemäß dem Stand der Technik weiter gereinigt, wobei das Lösungsmittel zurückgewonnen wurde.

### Beispiel 2 (erfindungsgemäß, isotherme Betriebsweise in Reaktor (2000) ohne Nachreaktor):

Als 42-massenprozentige Lösung in ortho-Dichlorbenzol wurden 5 kg/h 4,4'-Diaminodiphenylmethan (Strom (2)) mit 9,5 kg/h Phosgen als 54-massenprozentige Lösung in ortho-Dichlorbenzol (Strom (1)) mit einem rückgeführtem Carbaminsäurehaltigen Strom von 23,8 kg/h (Strom (3)) in einer Mischeinrichtung vereinigt, wobei die Konzentration an Carbaminsäurechlorid etwa 39% in ortho-Dichlorbenzol betrug. Es wurde ein stoffmengenbezogener Phosgenüberschuss auf das eingesetzte Amin von 5 % der Theorie dabei eingestellt **(Schritt (i)** des erfindungsgemäßen Verfahrens). Die Temperatur am Austritt der Mischeinrichtung betrug 139°C, und es herrschte ein Druck von 40 bar über Atmosphärendruck. Das Reaktionsgemisch gelang anschließend in einen beheizbaren turmförmigen Reaktor ((2000); **Schritt (ii)** des erfindungsgemäßen Verfahrens), dem zur Vermeidung von Thermalexpansionen stündlich 600 g Chlorwasserstoff als gasförmige Komponente am Kopf entnommen wurde **(Schritt (iii)** des erfindungsgemäßen Verfahrens). Der verbliebene flüssige Stoffstrom (Strom (6)) wurde anschließend mit einem massenbezogenen Verhältnis von 1,22 : 1 in zwei Ströme bei einer Temperatur von 140 °C aufgetrennt (**Schritt (iv)** des erfindungsgemäßen Verfahrens), wobei 19,5 kg/h (Strom (8)) des Stromes über mehrere Separatoren auf atmosphärischen Umgebungsdruck reduziert wurden (**Schritt (iv) (b)** des erfindungsgemäßen Verfahrens). Der andere Teil des Stromes wird wie oben beschrieben in die Mischeinrichtung zurückgeführt (**Schritt (iv) (a)** des erfindungsgemäßen Verfahrens). Die nach Durchlaufen der Separatoren erhaltene flüssige Roh-MMDI-Lösung, die kein MMDA mehr enthielt und einen Gehalt an Harnstoffgruppierungen enthaltenen Nebenkomponenten von 1,4 %, bezogen auf die Masse des in der Roh-MMDI-Lösung enthaltenen MMDI, aufwies, wurde destillativ gemäß dem Stand der Technik weiter gereinigt, wobei das Lösungsmittel zurückgewonnen wurde.

### Beispiel 3 (Vergleichsbeispiel):

Als Vergleich wird Beispiel 3 von DE-OS 1593412 herangezogen.

Das Vergleichsbeispiel ergab bei einem Phosgenüberschuss von 280 % der Theorie eine Ausbeute an MMDI von 94 %. In den Beispielen 1 und 2 wurde die Ausbeute an MMDI lediglich von Harnstoffgruppierungen enthaltenden Nebenkomponenten beeinträchtigt. Da der Gehalt an diesen die Qualität des MMDI schmälernden Nebenkomponenten gegenüber dem auf konventionelle, dem Stand der Technik entsprechende Weise hergestelltem MMDI in einem vergleichbaren Rahmen liegt, wird durch den erniedrigten Phosgenüberschuss und die geringere Menge an Lösungsmittel ein Vorteil hinsichtlich Sicherheit und Wirtschaftlichkeit erzielt.

## Patentansprüche

1. Kontinuierliches Verfahren zur Herstellung eines Polyisocyanats in der Flüssigphase durch Umsetzung des korrespondierenden Polyamins mit Phosgen, wobei als Zwischenstufen das entsprechende Carbaminsäurechlorid und das entsprechende Aminhydrochlorid auftreten, bei dem
(i) ein Phosgen-haltiger Strom (1), ein Polyamin-haltiger Strom (2) und ein Carbaminsäurechlorid- und Aminhydrochlorid-haltiger Strom (3) in einer Mischeinrichtung (1000) vermischt werden, wobei Phosgen in einem stöchiometrischen Überschuss von > 0 % bis 50 % der Theorie, bezogen auf die Amingruppen des in Strom (2) enthaltenen Polyamins, eingesetzt wird,
(ii) der in Schritt (i) erhaltene Mischstrom (4) durch einen Reaktor (2000), in dem ein absoluter Druck von 20 bar bis 60 bar und eine Temperatur von 80 °C bis 200 °C herrschen, geführt wird, wobei ein Carbaminsäurechlorid- und Aminhydrochlorid-haltiger Strom (5) gebildet wird,
(iii) optional ein gasförmiger Purge-Strom (9) aus dem in Schritt (iii) gebildeten Carbaminsäurechlorid- und Aminhydrochlorid-haltigen Strom (5) ausgeschleust wird, wobei ein an gasförmigen Anteilen abgereicherter Carbaminsäurechlorid- und Aminhydrochlorid-haltiger Strom (6) erhalten wird,
(iv) der in Schritt (iii) erhaltene Strom (5) oder der in Schritt (iv) erhaltene Strom (6) in zwei Teilströme (7, 8) getrennt wird, wobei
(a) der Teilstrom (7) als Carbaminsäurechlorid- und Aminhydrochlorid-haltiger Strom (3) in Schritt (i) eingesetzt wird und
(b) der Teilstrom (8) zum gewünschten Polyisocyanat umgesetzt wird.

2. Verfahren nach Anspruch 1, bei dem der Reaktor (2000) in Schritt (ii) adiabat betrieben wird.

3. Verfahren nach Anspruch 1, bei dem der Reaktor (2000) in Schritt (ii) isotherm betrieben wird.

4. Verfahren nach einem der Ansprüche 1 bis 3, bei dem in Schritt (iv) (b) der Carbaminsäurechlorid- und Aminhydrochlorid-haltige Strom (8) zunächst
(1) durch einen isotherm betriebenen Reaktor (5100)
und dann
(2) durch eine Kaskade an Abscheidern mit sukzessiv abnehmendem Druck
geführt wird.

5. Verfahren nach Anspruch 3, bei dem in Schritt (iv) (b) der Carbaminsäurechlorid- und Aminhydrochlorid-haltige Strom (8) direkt durch eine Kaskade an Abscheidern mit sukzessiv abnehmendem Druck geführt wird.

6. Verfahren nach einem der Ansprüche 1 bis 5, bei dem Schritt (iv) (b) ohne Zugabe von Phosgen erfolgt.

7. Verfahren nach einem der Ansprüche 1 bis 6, bei dem
der Phosgen-haltige Strom (1) einen Massenanteil von 10 % bis 90 % an Phosgen und einen Massenanteil von 90 % bis 10 % eines inerten Lösungsmittels umfasst, jeweils bezogen auf die Gesamtmasse des Stroms (1),
der Polyamin-haltige Strom (2) einen Massenanteil von 10 % bis 50 % an Polyamin und einen Massenanteil von 90 % bis 50 % eines inerten Lösungsmittels umfasst, jeweils bezogen auf die Gesamtmasse des Stroms (2).

8. Verfahren nach Anspruch 7, bei dem das inerte Lösungsmittel in Strom (1) und in Strom (2) unabhängig voneinander ausgewählt ist aus der Gruppe bestehend aus Chlorbenzol, Dichlorbenzol, Toluol, Dioxan, Dimethylsulfoxid und einer Mischung aus zwei oder mehr der vorgenannten Lösungsmittel.

9. Verfahren nach einem der Ansprüche 1 bis 8, bei dem das Verhältnis der Stoffmengen von Carbaminsäurechlorid und Aminhydrochlorid einerseits zu Polyisocyanat andererseits in Strom (5) 2 : 1 bis 100 : 1 beträgt.

10. Verfahren nach einem der Ansprüche 1 bis 9, bei dem die in Schritt (i) eingesetzte Mischeinrichtung ein dynamischer Mischer ist.

11. Verfahren nach einem der Ansprüche 1 bis 10, bei dem in Schritt (i) Phosgen in einem stöchiometrischen Überschuss von > 0 % bis 20 % der Theorie, bezogen auf die Amingruppen des in Strom (2) enthaltenen Polyamins, eingesetzt wird.

12. Verfahren nach Anspruch 11, bei dem in Schritt (i) Phosgen in einem stöchiometrischen Überschuss von > 0 % bis 15 % der Theorie, bezogen auf die Amingruppen des in Strom (2) enthaltenen Polyamins, eingesetzt wird.

13. Verfahren nach Anspruch 12, bei dem in Schritt (i) Phosgen in einem stöchiometrischen Überschuss von > 0 % bis 10 % der Theorie, bezogen auf die Amingruppen des in Strom (2) enthaltenen Polyamins, eingesetzt wird.

14. Verfahren nach einem der Ansprüche 1 bis 13, bei dem nicht umgesetztes Phosgen mittels Kondensation durch indirekte Kühlung zurückgewonnen wird.

15. Verfahren nach einem der Ansprüche 1 bis 14, bei dem das Isocyanat ausgewählt ist aus der Gruppe bestehend aus Methylendiphenyldiisocyanat als reines Isomer oder als Isomerengemisch, Polymethylenpolyphenylpolyisocyanat, Gemische aus Methylendiphenyldiisocyanat und Polymethylenpolyphenylpolyisocyanat, Toluylendiisocyanat als reines Isomer oder Isomerengemisch, Isomere des Xylylendiisocyanats, Isomere des Diisocyanatobenzols, 2,6-Xylolisocyanat und 1,5-Naphthylendiisocyanat.

## Claims

1. Continuous process for preparing a polyisocyanate in the liquid phase by reaction of the corresponding polyamine with phosgene, with the corresponding carbamoyl chloride and the corresponding amine hydrochloride occurring as intermediates, wherein
(i) a phosgene-containing stream (1), a polyamine-containing stream (2) and a stream (3) containing carbamoyl chloride and amine hydrochloride are mixed in a mixing device (1000), where phosgene is used in a stoichiometric excess of from > 0% to 50% of theory, based on the amine groups of the polyamine present in stream (2)
(ii) the mixed stream (4) obtained in step (i) is conveyed through a reactor (2000) in which an absolute pressure of from 20 bar to 60 bar and a temperature of from 80°C to 200°C, prevail, forming a stream (5) containing carbamoyl chloride and amine hydrochloride
(iii) a gaseous purge stream is optionally discharged from the stream (5) containing carbamoyl chloride and amine hydrochloride which is formed in step (ii), giving a stream (6) which contains carbamoyl chloride and amine hydrochloride and has been depleted in gaseous components,
(iv) the stream (5) obtained in step (ii) or the stream (6) obtained in step (iii) is divided into two substreams (7, 8), where
(a) the substream (7) is used as stream (3) containing carbamoyl chloride and amine hydrochloride in step (i) and
(b) the substream (8) is converted into the desired polyisocyanate.

2. Process according to Claim 1, wherein the reactor (2000) in step (ii) is operated adiabatically.

3. Process according to Claim 1, wherein the reactor (2000) in step (ii) is operated isothermally.

4. Process according to any of Claims 1 to 3, wherein, in step (iv) (b), the stream (8) containing carbamoyl chloride and amine hydrochloride is firstly conveyed
(1) through an isothermally operated reactor (5100) and then
(2) through a cascade of separators with gradually decreasing pressure.

5. Process according to Claim 3, wherein, in step (iv) (b), the stream (8) containing carbamoyl chloride and amine hydrochloride is conveyed directly through a cascade of separators with gradually decreasing pressure.

6. Process according to any of Claims 1 to 5, wherein step (iv) (b) is carried out without addition of phosgene.

7. Process according to any of Claims 1 to 6, wherein
the phosgene-containing stream (1) comprises a proportion by mass of from 10% to 90% of phosgene and a proportion by mass of from 90% to 10% of an inert solvent, in each case based on the total mass of the stream (1),
the polyamine-containing stream (2) comprises a proportion by mass of from 10% to 50% of polyamine and a proportion by mass of from 90% to 50% of an inert solvent, in each case based on the total mass of the stream (2).

8. Process according to Claim 7, wherein the inert solvent in stream (1) and in stream (2) is selected independently from the group consisting of chlorobenzene, dichlorobenzene, toluene, dioxane, dimethyl sulfoxide and a mixture of two or more of the abovementioned solvents.

9. Process according to any of Claims 1 to 8, wherein the ratio of the molar amounts of carbamoyl chloride and amine hydrochloride to polyisocyanate in stream (5) is from 2 : 1 to 100 : 1.

10. Process according to any of Claims 1 to 9, wherein the mixing device used in step (i) is a dynamic mixer.

11. Process according to any of Claims 1 to 10, wherein, in step (i), phosgene is used in a stoichiometric excess of from > 0 % to 20% of theory, based on the amine groups of the polyamine present in stream (2).

12. Process according to Claim 11, wherein, in step (i), phosgene is used in a stoichiometric excess of from > 0% to 15% of theory, based on the amine groups of the polyamine present in stream (2).

13. Process according to Claim 12, wherein, in step (i), phosgene is used in a stoichiometric excess of from > 0% to 10% of theory, based on the amine groups of the polyamine present in stream (2).

14. Process according to any of Claims 1 to 13, wherein unreacted phosgene is recovered by means of condensation by indirect cooling.

15. Process according to any of Claims 1 to 14, wherein the isocyanate is selected from the group consisting of methylenedi(phenyl isocyanate) as pure isomer or as isomer mixture, polymethylenepolyphenyl polyisocyanate, mixtures of methylenedi(phenyl - diisocyanate) and polymethylenepolyphenyl polyisocyanate, tolylene diisocyanate as pure isomer or isomer mixture, isomers of xylylene diisocyanate, isomers of diisocyanatobenzene, xylene 2,6-diisocyanate and naphthalene 1,5-diisocyanate.

## Revendications

1. Procédé continu de fabrication d'un polyisocyanate dans la phase liquide par mise en réaction de la polyamine correspondante avec du phosgène, le chlorure de l'acide carbamique correspondant et le chlorhydrate d'amine correspondant étant formés en tant qu'intermédiaires, selon lequel
(i) un courant contenant du phosgène (1), un courant contenant la polyamine (2) et un courant contenant du chlorure de l'acide carbamique et du chlorhydrate d'amine (3) sont mélangés dans un dispositif de mélange (1000), le phosgène étant utilisé en un excès stoechiométrique de > 0 % à 50 % de la théorie, par rapport aux groupes amino de la polyamine contenue dans le courant (2),
(ii) le courant de mélange (4) obtenu à l'étape (i) est acheminé au travers d'un réacteur (2000), dans lequel règnent une pression absolue de 20 bar à 60 bar et une température de 80 °C à 200 °C, un courant (5) contenant du chlorure de l'acide carbamique et du chlorhydrate d'amine étant formé,
(iii) un courant de purge gazeux (9) est éventuellement évacué à partir du courant (5) contenant du chlorure de l'acide carbamique et du chlorhydrate d'amine formé, un courant (6) contenant du chlorure de l'acide carbamique et du chlorhydrate d'amine appauvri en fractions gazeuses étant obtenu,
(iv) le courant (5) obtenu à l'étape (iii) ou le courant (6) obtenu à l'étape (iv) est séparé en deux courants partiels (7, 8),
(a) le courant partiel (7) étant utilisé en tant que courant contenant du chlorure de l'acide carbamique et du chlorhydrate d'amine (3) à l'étape (i), et
(b) le courant partiel (8) étant transformé en le polyisocyanate souhaité.

2. Procédé selon la revendication 1, selon lequel le réacteur (2000) à l'étape (ii) est exploité en mode adiabatique.

3. Procédé selon la revendication 1, selon lequel le réacteur (2000) à l'étape (ii) est exploité en mode isotherme.

4. Procédé selon l'une quelconque des revendications 1 à 3, selon lequel, à l'étape (iv)(b), le courant (8) contenant du chlorure de l'acide carbamique et du chlorhydrate d'amine est acheminé tout d'abord
(1) au travers d'un réacteur exploité en mode isotherme (5100),
puis
(2) au travers d'une cascade de séparateurs à pression successivement décroissante.

5. Procédé selon la revendication 3, selon lequel, à l'étape (iv)(b), le courant (8) contenant du chlorure de l'acide carbamique et du chlorhydrate d'amine est acheminé directement au travers d'une cascade de séparateurs à pression successivement décroissante.

6. Procédé selon l'une quelconque des revendications 1 à 5, selon lequel l'étape (iv)(b) a lieu sans ajout de phosgène.

7. Procédé selon l'une quelconque des revendications 1 à 6, selon lequel
le courant contenant du phosgène (1) comprend une proportion en masse de 10 % à 90 % de phosgène et une proportion en masse de 90 % à 10 % d'un solvant inerte, à chaque fois par rapport à la masse totale du courant (1),
le courant contenant la polyamine (2) comprend une proportion en masse de 10 % à 50 % de polyamine et une proportion en masse de 90 % à 50 % d'un solvant inerte, à chaque fois par rapport à la masse totale du courant (2) .

8. Procédé selon la revendication 7, selon lequel le solvant inerte dans le courant (1) et dans le courant (2) sont choisis indépendamment l'un de l'autre dans le groupe constitué par le chlorobenzène, le dichlorobenzène, le toluène, le dioxane, le diméthylsulfoxyde et un mélange de deux ou plus des solvants susmentionnés.

9. Procédé selon l'une quelconque des revendications 1 à 8, selon lequel le rapport entre les quantités de matière de chlorure de l'acide carbamique et de chlorhydrate d'amine d'une part et de polyisocyanate d'autre part dans le courant (5) est de 2:1 à 100:1.

10. Procédé selon l'une quelconque des revendications 1 à 9, selon lequel le dispositif de mélange utilisé à l'étape (i) est un mélangeur dynamique.

11. Procédé selon l'une quelconque des revendications 1 à 10, selon lequel, à l'étape (i), le phosgène est utilisé en un excès stoechiométrique de > 0 % à 20 % de la théorie, par rapport aux groupes amino de la polyamine contenue dans le courant (2).

12. Procédé selon la revendication 11, selon lequel, à l'étape (i), le phosgène est utilisé en un excès stoechiométrique de > 0 % à 15 % de la théorie, par rapport aux groupes amino de la polyamine contenue dans le courant (2).

13. Procédé selon la revendication 12, selon lequel, à l'étape (i), le phosgène est utilisé en un excès stoechiométrique de > 0 % à 10 % de la théorie, par rapport aux groupes amino de la polyamine contenue dans le courant (2).

14. Procédé selon l'une quelconque des revendications 1 à 13, selon lequel le phosgène non réagi est récupéré par condensation par refroidissement indirect.

15. Procédé selon l'une quelconque des revendications 1 à 14, selon lequel l'isocyanate est choisi dans le groupe constitué par le diisocyanate de diphénylméthylène sous la forme d'un isomère pur ou sous la forme d'un mélange d'isomères, le polyisocyanate de polyphényl-polyméthylène, les mélanges de diisocyanate de diphénylméthylène et de polyisocyanate de polyphényl-polyméthylène, le diisocyanate de toluylène sous la forme d'un isomère pur ou sous la forme d'un mélange d'isomères, les isomères de diisocyanate de xylylène, les isomères de diisocyanatobenzène, l'isocyanate de 2,6-xylène et le diisocyanate de 1,5-naphtylène.
